(19) Europäisches Patentamt / European Patent Office / Office européen des brevets

(11) EP 4 462 095 B1

(12) **EUROPEAN PATENT SPECIFICATION**

(45) Date of publication and mention
of the grant of the patent:
**25.02.2026 Bulletin 2026/09**

(21) Application number: **23315180.2**

(22) Date of filing: **10.05.2023**

(51) International Patent Classification (IPC):
*G01M 3/32* *(2006.01)*        *G01K 7/42* *(2006.01)*

(52) Cooperative Patent Classification (CPC):
**G01M 3/3236; G01K 7/42; G01K 15/00;
G01K 15/005**

(54) **METHOD FOR DETERMINING A QUANTITY OF GAS CONTAINED IN AN INSULATED SWITCHGEAR**

VERFAHREN ZUR BESTIMMUNG EINER IN EINER ISOLIERTEN SCHALTANLAGE
ENTHALTENEN GASMENGE

PROCÉDÉ DE DÉTERMINATION D'UNE QUANTITÉ DE GAZ CONTENUE DANS UN
APPAREILLAGE DE COMMUTATION ISOLÉ

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**13.11.2024 Bulletin 2024/46**

(73) Proprietor: **SCHNEIDER ELECTRIC INDUSTRIES
SAS
92500 Rueil-Malmaison (FR)**

(72) Inventors:
• **Alberto, Diego
38700 CORENC (FR)**
• **Boguslawski, Bartosz
38100 GRENOBLE (FR)**

(74) Representative: **Plasseraud IP
104 Rue de Richelieu
CS92104
75080 Paris Cedex 02 (FR)**

(56) References cited:
JP-A- H08 105 813        US-A1- 2015 308 938
US-A1- 2017 030 799

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

This disclosure relates to medium voltage electrical distribution systems, and more particularly to a method for determining the quantity of the gas contained in the tank of a gas insulated switchgear. In addition, this method of gas quantity determination can serve as a basis for a tank leak detection method.

## Background Art

Gas insulated switchgears include a connection/disconnection element for one or several phases of a medium voltage electrical network. This connection/disconnection element is installed in a tank filled with gas. The dielectric properties of the gas help to assist the arc switching or to provide electrical insulation between the different phases of the electrical network. The tank and all its interfaces are sealed, so that the tank doesn't need to be replenished throughout the useful life of the equipment. The gas contained in the tank may for example be an inert gas like sulfur hexafluoride $SF_6$. In other applications, the gas can also be pressurized air.

It is known to monitor the quantity of the gas contained in the tank by measuring its temperature and pressure. Prior art documents describing such monitoring are disclosed in US2015/308938 A1, JP H08105813A and US 2017/030799 A1. For this, a sensor with a pressure sensing probe and a temperature sensing probe is fixed in an interface plug fixed to a tank panel. The sensing elements are thus in contact with the gas inside the tank. The quantity of the gas can therefore be calculated and monitored during the operation of the switchgear from the pressure and temperature measurements. The correct operation of the electrical equipment can be checked from this quantity monitoring. For example, a drop in gas quantity may indicate a premature leakage of the tank, that may require maintenance operation for correction.

The detection of a small leak can be difficult. A small leak causes by its very nature a slow decrease in gas quantity. For example, a pressure drop from 1.2 bar to 1.1 bar can take several weeks or months and still needs to be detected. In addition, the pressure inside the tank is influenced by the temperature of the gas, which is itself influenced by the ambient temperature, and by the intensity of the current in the electrical conductors. Internal pressure and temperature of the tank are therefore influenced by the operating conditions of the electrical equipment. As the gas pressure and temperature sensors are located on the external walls of the tank, the measured temperature may not be representative of the real gas temperature. In addition, the temperature of the gas contained in the tank may not be homogeneous and temperature gradients may exist. Therefore, the accuracy of the gas quantity calculation required to detect a low level of leakage may be difficult to achieve.

The present disclosure proposes a method for estimating gas quantity in a gas-filled tank with an improved accuracy. A leakage detection method using the method for estimating gas quantity is also proposed.

## Summary

To this end, it is proposed a method for determining a quantity of a gas contained in a tank of a gas insulated switchgear, the tank comprising an embedded gas temperature sensor and an embedded gas pressure sensor, the method according to appended claim 1.

During the calibration phase, whose duration is negligible compared to the expected service life of the switchgear, the tank is considered to be perfectly sealed, and the quantity of gas contained in the tank is therefore assumed to be constant. Possible discrepancies between the gas quantities determined for the successive calibration samples don't come from any real leakage, but come from the fact that the measured gas temperature is not fully representative of the actual gas temperature inside the tank. Therefore, a corrected gas temperature can be determined by applying a constraint that the determined gas quantity has to be identical for all the set of samples that are acquired during the calibration phase. The corrected gas temperature is the temperature for which the gas quantity predicted by the gas state equation is identical for the all the sets of calibration samples, or at least for which the discrepancies between the gas quantities are minimal. Once the model for determination of this corrected gas temperature has been determined, the same model is also applied during the measurement phase. Using this gas temperature model increases the accuracy of the gas quantity calculation compared to a method based only on the raw measurements of the different sensors. As the model is extracted from a limited number of experimental data, it is easy to calibrate and can quickly be adapted to any gas tank / temperature sensors configurations. The method doesn't need any additional sensor to be fitted during the calibration phase, therefore the electrical equipment in which the tank is integrated can remain in its original configuration without any modification, even temporary. The calibration phase, also called learning phase, can be carried out when the gas insulated switchgear is in new condition and is commissioned for being put in service. The calibration phase can also be repeated after every major maintenance operation performed on the equipment.

The following features can optionally be implemented, separately or in combination one with the others:

The measurement phase follows the calibration phase. In other words, the calibration phase is performed before the measurement phase is carried out.

The measurement phase is a monitoring phase that may be carried out throughout the service life of the switchgear.

The measurement phase may be carried out continuously.

Alternatively, the measurement phase may be carried out at different time intervals.

The calibration phase comprises a sub-step of checking of the tank tightness.

At each acquisition of a set of samples, the gas pressure measured by the embedded gas pressure sensor, the gas temperature measured by the embedded gas temperature sensor and the ambient temperature measured by an ambient temperature sensor outside the tank are acquired simultaneously.

The volume of the tank is constant.

The quantity of the gas encompasses closely related physical parameters that may be the gas number of moles, or the gas mass. It also encompasses the gas density since the internal volume of the tank is kept constant.

At step (iii), the model may be updated so that the gas quantity determined for each set of samples is the same.

The first plurality of successive sets of calibration samples comprises at least three samples.

In an embodiment, the calibration phase comprises the steps :

(iii-a1) if at step (iii) the difference between the gas quantity determined for each set of calibration samples is inferior to a first predefined threshold, end the calibration phase,

(iii-a2) if at step (iii) the difference between the gas quantity determined for each set of calibration samples is superior to equal to the first predefined threshold, add one extra set of calibration samples to the second plurality of sets of calibration samples to obtain an updated second plurality of sets of calibration samples and iterate steps (ii) and (iii) with the updated second plurality of sets of calibration samples.

The calibration phase ends when the expected accuracy is obtained. As long as the expected accuracy is not yet obtained, some calibration samples are added to the second plurality of samples, which means that more samples are used to calibrate the model. As long as this targeted accuracy is not obtained, more calibration data are added to improve the model.

In an embodiment, the method comprises the step :

(iii-a3) if at step (iii-a2) the difference between the gas quantity determined for the first plurality of calibration samples is superior or equal to the first predefined threshold, iterate step (i) to acquire at least one extra set of calibration samples and obtain an updated first plurality of successive sets of calibration samples, and iterate steps (ii) and (iii) to update the corrected gas temperature model.

In other words, samples are added to the second plurality of sets of samples until all calibration samples of the first plurality has been integrated, meaning that the second plurality is identical to the first plurality. If the determined difference is still superior or equal to the predefined acceptance threshold after all calibration samples have been used for calibrating the model, some extra sets of samples are acquired to continue the calibration process with more set of samples in order to reach the expected accuracy.

Alternatively, the calibration process can be restarted from scratch with a completely new set of samples.

In an embodiment, the method comprises between the calibration phase and the measurement phase a validation phase of the corrected gas temperature model, the validation phase comprising the steps :

(iii-b1) for a third plurality of sets of calibration samples comprised in the first plurality of sets of calibration samples, determine a gas quantity contained in the tank from the gas state equation and from the corrected gas temperature model based on the acquired gas temperature, the acquired gas pressure and the acquired ambient temperature,

(iii-b2) determine a difference between the gas quantities determined for each set of samples of the third plurality of sets of calibration samples,

(iii-b3) if the determined difference is inferior to a second predefined threshold, authorize entry into the measurement phase.

Once the calibration acceptance criterion is met, and the calibration phase is ended, an intermediate validation phase may be carried out before entering in the measurement phase. For this, some set of samples of the initially acquired data may be used for checking that the associated determined gas quantities are close enough to the expected results.

The third plurality of sets of calibration samples may have no common element with the second plurality of sets of calibration samples.

In other words, the model may be calibrated using certain sets of samples, and may be validated using different set of samples. A same set of samples is not used for both calibration and validation of the gas corrected temperature model.

In an embodiment, the method comprises the step:

(iii-b4) if the determined difference is superior or equal to the second predefined threshold, iterate step (i) to acquire at least one extra set of calibration samples and iterate steps (ii) and (iii) to update the corrected gas temperature model.

In case the accuracy criterion for accepting the model is still not met when all the available data initially acquired have been used, some extra data acquisition is performed. For this, step (i) is iterated to add at least one more set of samples, and the gas corrected temperature model M is updated with this extra set of samples.

The second predefined threshold may be higher than the first predefined threshold .

The second predefined threshold may be equal to the first predefined threshold .

According to an embodiment of the proposed method, the gas state equation is the ideal gas law.

The state equation is, for the ideal gas model:

$$P * V = n * r * T$$

(equation 1)

According to another embodiment of the proposed method, the gas state equation is a real gas law.

For example, Van der Waals state equation can be employed. The Virial state equation can also be used. Other different real gas laws could also be used.

In an embodiment of the method, the corrected temperature model is a multivariate linear regression model.

For example, the corrected gas temperature is a linear combination of the acquired gas pressure, of the acquired gas temperature and of the acquired ambient temperature. The corrected gas temperature satisfies :

$$Tcor = K_0 + K_1 * P + K_2 * T + K_3 * TA$$

(equation 2)

with Tcor the corrected gas temperature, P the acquired gas pressure, T acquired gas temperature and TA the acquired ambient temperature, and

with $K_0$, $K_1$, $K_2$, $K_3$ constant coefficients.

When the number of acquired samples is smaller than the number of measured variables, the parameters of such an underdetermined linear system may be derived thanks to the minimum L2 norm solution using, for example, the method of Lagrange multiplier. On the contrary, if the number of samples is larger than the number of measured variables, a classic regression method, as the Least Squares regression method can be chosen.

In another embodiment of the method, the corrected temperature model is a perceptron model.

The acquired gas pressure, the acquired gas temperature and the acquired ambient temperature are inputs of the perceptron. The corrected temperature is the output of the perceptron.

In yet another embodiment of the method, the corrected temperature model is a support vector regression.

In yet another example of implementation of the method, the corrected temperature model is a gradient boosting regression.

A time interval between two successive sets of calibration samples of the first plurality of successive sets of calibration samples may be more than one minute and less than one hour.

In an embodiment of the method, the step (i) of acquiring the first plurality of successive sets of calibration samples may comprise the sub-step :

- after the acquisition of one sample and before the acquisition of the next sample, modifying an electrical current intensity in the switchgear so that the temperature of the gas contained in the tank is modified.

By spreading the values of the set of calibration samples, the robustness of the model of determination of corrected gas temperature can be improved, as the measurements are less subject to noise and to the influence of the environmental conditions.

The disclosure also relates to a method for detecting a gas leakage in a tank of a gas insulated switchgear, comprising the steps:

- determining the quantity of the gas contained in the tank by the method as described earlier,
- detecting a gas leakage based on the evolution of the gas quantity in function of time.

The gas leakage detection method may comprise the steps :

- determining an initial quantity of the gas contained in the tank at the end of the calibration phase,
- determining an updated quantity of the gas contained in the tank during the measurement phase,
- determining a difference between the determined initial quantity and the determined updated quantity,
- if the determined difference is higher than a predetermined threshold, detecting that a leakage is present,
- if the determined difference is lower than or equal to the predetermined threshold, detecting that the tank is sealed.

In an embodiment, the determined difference is an absolute difference.

In this case, the predetermined threshold is an absolute threshold.

In another embodiment, the determined difference may be a relative difference.

In this case, the predetermined threshold is a relative threshold.

The method may comprise the step:

- emitting a warning signal if a leakage is detected.

The disclosure also relates to an electrical equipment according to appended claim 15.

**Brief Description of Drawings**

Other features, details and advantages will be shown in the following detailed description and on the figures, on which:

- Figure 1 is a schematic view of an electrical equipment comprising a gas insulated switchgear contained in a tank,

- Figure 2 is a temporal curve illustrating the evolution of the internal temperature of the gas contained in the tank,

- Figure 3 is a temporal curve illustrating the evolution of the pressure of the gas contained in the tank,

- Figure 4 is a temporal curve illustrating the evolution of the corrected gas temperature determined for the gas contained in the tank,

- Figure 5 is a temporal curve illustrating the evolution of the determined quantity of gas contained in the tank, when the tank is fluid tight,

- Figure 6 is another temporal curve illustrating the evolution of the determined quantity of gas contained in the tank, when a tank leakage is present,

- Figure 7 is a block diagram of different steps of an embodiment of the proposed method,

- Figure 8 is a temporal curve illustrating some aspects of the proposed method.

**Description of Embodiments**

In order to make the figures easier to read, the various elements are not necessarily represented to scale. In these figures, identical elements receive the same reference number. Certain elements or parameters can be indexed, that is to say designated for example by 'first element' or second element, or first parameter and second parameter, etc. The purpose of this indexing is to differentiate elements or parameters that are similar, but not identical. This indexing does not imply a priority of one element, or one parameter over another, and their names can be interchanged. When it is mentioned that a subsystem comprises a given element, the presence of other elements in this subsystem is not excluded.

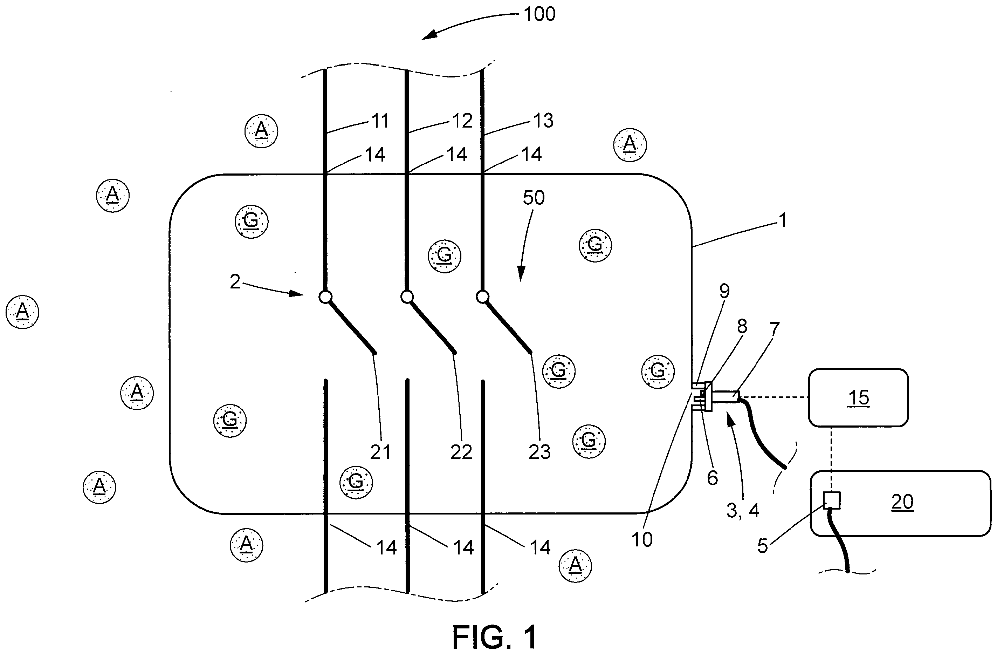

Figure 1 schematically illustrates an electrical equipment 100 comprising a switchgear 2, a tank 1 configured for accommodating the switchgear 2, and an electronic control unit 15. The electronic control unit 15 is configured for implementing a gas quantity determination method that will be described below. The electronic control unit 15 is also configured for implementing a gas leakage detection method that will also be described in detail below.

The tank 1 defines an enclosure configured to accommodate the switchgear 2. The tank 1 comprises several panels assembled to form a sealed enclosure defining a sealed volume. For example, the panels of the tank 1 are welded together. The panels may be fixed on a subframe that ensures a stiffness sufficient to resist without noticeable deformation to the pressure differential between the inside and the outside of the tank 1. The volume of the tank 1 is thus constant throughout its service life.

The tank 1 comprises sealed interfaces 14 allowing the entry and exit of three electrical conductors 11, 12, 13 connected to the switchgear 2. Each of the three electrical conductors 11, 12, 13 corresponds to a different phase of an electrical circuit. Each phase of the electrical circuit can be respectively interrupted by an interrupter 21, 22, 23. On figure 1, the switchgear 2 is represented in an opened position of the interrupters 21, 22, 23 in which the current is interrupted.

The inside volume of the tank 1 is filled with a pressurized gas G when the tank 1 is in normal operating conditions. The pressurized gas G avoids the creation of an electric arc when the switchgear 2 is operated to interrupt the electrical current in the circuit. The outside of the tank 1 is exposed to the temperature and the pressure of the ambient air A of the room in which the tank 1 is installed. The pressure of the gas within the tank 1 is higher than the ambient pressure outside of the tank 1, which is the local atmospheric pressure. The tank 1 may be filled with an inert gas like sulfur hexafluoride ($SF_6$). The tank 1 may also be filled with pressurized air.

Although the tank 1 is built to be fluid tight, small leakages may occur during the lifetime of the electrical equipment 100 and the quantity of gas G contained within the internal volume of the tank 1 may be slowly reduced. The dielectric properties thus slowly deteriorate and the risks of creating an electric arc during operation of the equipment increase. For this reason, the quantity N of the gas contained in the tank 1 is monitored so that the operators of the electrical equipment 100 can take adequate corrective actions in case the gas quantity N becomes insufficient for a safe operation of the electrical equipment 100. This gas quantity monitoring is based on gas temperature and gas pressure measurements. As previously stated, the gas quantity N can be gas mass, or gas number of moles. It can also be gas density, since the internal volume of the tank is kept constant. Conversion from one parameter selected from mass, number of moles, density to another of these selected parameters can be made by a multiplicative factor. These three parameters can thus be considered equivalent physical parameters.

The tank 1 is equipped with an embedded gas temperature sensor 3.

The embedded temperature sensor 3 comprises a temperature sensitive element 6 configured for delivering an electric signal representative of the temperature of the sensitive element 6. The temperature sensitive element 6 of the embedded temperature sensor 3 is located inside the tank 1 and is in contact with the gas G contained in the tank 1.

The temperature sensing element 6 of the embedded gas temperature sensor 3 may comprise a thermistor, for instance a negative coefficient thermistor. In a variant, the temperature sensing element 6 may comprise a thermocouple.

The embedded temperature sensor 3 comprises a sensor body 7 in contact with the ambient air A outside the tank 1. The sensor body 7 protrudes from an external side of a panel of the tank 1. The implementation of the sensor is easy, since it is disposed outside the tank 1, and its connection wires are kept outside the internal volume of the tank 1, therefore don't interference with the tank 1 tightness.

The embedded temperature sensor 3 is attached to an interface plug 9 fixed to a panel of the tank 1. The interface plug 9 is inserted in a hole formed in a panel and for example welded to the panel along the periphery of the interface plug. The interface plug 9 defines an opening 10 in a panel of the tank 1, through which the sensitive element 6 can be in contact with the gas G inside the tank 1.

The interface plug 9 may comprise an internal thread for fixing the sensor body 7. The opening 10 is obturated by the body 7 of the gas temperature sensor 3 when the gas temperature sensor 3 is fitted. A seal, not represented, may be disposed between the interface plug 9 and the body 7 of the temperature sensor to ensure fluid tightness of the tank 1.

The tank 1 is also equipped with an embedded pressure sensor 4.

The embedded pressure sensor 4 comprises a pressure sensitive element 8 configured for delivering an electric signal representative of the pressure applied on the sensitive element. The sensitive element 8 of the embedded pressure sensor 4 is located inside the tank 1 and is in contact with the gas G contained in the tank 1.

The pressure sensing element 8 and the temperature sensing element 6 may be housed within the same sensor body 7, as it is the case on the illustrated example. A single interface plug 9 is used to install the two sensors and obtain gas temperature measurement and gas pressure measurement. The sensor is thus a multi-function sensor combining pressure measurement and temperature measurement.

According to a non-represented design variant, the embedded pressure sensor 4 may be a dedicated sensor, separated and independent from the gas temperature sensor 3. In this case, the pressure sensor 4 is fixed to another interface plug defining another opening hole in a panel of the tank 1.

The electrical equipment 100 comprises an ambient temperature sensor 5. The ambient temperature sensor 5 comprises a sensitive element configured for delivering an electric signal representative of the temperature of the sensitive element. The sensitive element of the ambient temperature sensor 5 is located outside the tank 1 and is in contact with the ambient air outside the tank 1. The ambient temperature sensor 5 is for example installed on a control panel 20 of the switchgear 2. The ambient temperature sensor 5 and the external surface of the tank 1 are spaced apart from each other. The distance between the ambient temperature sensor 5 and the external surface of the tank 1 is preferably larger than 30 centimeters. The ambient temperature measurement is thus not affected by the heat dissipated by the tank 1. The measurement of the ambient temperature sensor 5 is considered to be fully representative of the ambient temperature of the room in which the tank 1 is installed.

The ambient temperature sensor 5 may comprise a thermistor, like for example a negative coefficient thermistor. The ambient temperature sensor 5 may comprise a thermocouple.

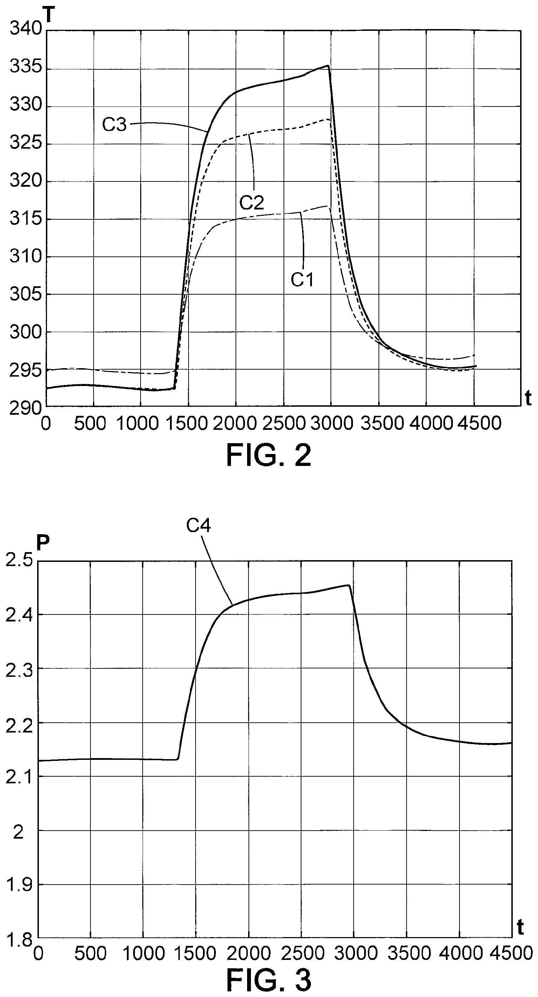

Figure 2 illustrates the evolution of the internal temperature of the gas contained in the tank, measured by different methods.

Curve C1 illustrates the temperature measured by the embedded temperature sensor 3. Curve C2 illustrates the average temperature measured by a group of sensors fitted inside the tank 1. For example, one sensor is fitted near the top of the tank 1, one other sensor is fitted in the middle of the tank, and another sensor is fitted close to the bottom of tank 1. These sensors have been installed only for experimentation and are not part of the electrical equipment 100 when it is in regular operation.

The two curves C1 and C2 are offset, indicating that the average temperature of the gas measured at different locations of the tank 1 is not the same as the temperature measured by the sensor fitted to a panel of the tank 1. One reason is that the electrical conductors 11, 12, 13 dissipate heat, while no forced gas circulation takes place. Therefore, some temperature gradients exist within the tank 1.

Furthermore, the temperature of the gas contained in the tank 1 changes over time in function of the power required by the electrical network, because of the variable heat dissipated by the electrical currents circulating inside the tank 1 enclosure. On figure 2, the current intensity increases at instant 1400 minutes, and decrease at instant 3000 minutes. These current intensity variations cause the temperature to increase and then decrease.

Figure 3 illustrates the evolution of the pressure of the gas contained in the tank, measured by the embedded pressure sensor 4. Because of the temperature changes, the pressure of the gas also changes over time. Temperature curve and pressure curve have similar shapes.

The pressure of the gas contained in the tank 1 can be considered as homogeneous. The pressure gradient between different locations of the tank 1 is negligible.

The proposed method is for determining a quantity N of a gas G contained in a tank 1 of a gas insulated switchgear 2, the tank 1 comprising an embedded gas temperature sensor 3 and an embedded gas pressure sensor 4.

The method comprises the following steps during a calibration phase:

(i) acquiring a first plurality n of successive sets of calibration samples $S_1$, $S_2$, ..., $S_n$, each set of calibration samples $S_1$, $S_2$,..., $S_n$ comprising a gas pressure $P_1$, $P_2$, ..., $P_n$ measured by the embedded gas pressure sensor 4, a gas temperature $T_1$, $T_2$, ..., $T_n$ measured by the embedded gas temperature sensor 3 and an ambient temperature $TA_1$, $TA_2$, ..., $TA_n$ measured by an ambient temperature sensor 5 outside the tank 1,

(ii) for a second plurality p of sets of calibration samples $S_1$, $S_2$, ..., $S_p$ comprised in the first plurality n of sets of calibration samples $S_1$, $S_2$, ..., $S_n$, determine a gas quantity $N_1$, $N_2$, ...,$N_p$ contained in the tank 1 from a gas state equation E based on the acquired gas pressure $P_1$, $P_2$, ...,$P_p$, and on a corrected gas temperature model M based on the acquired gas temperature $T_1$, $T_2$, ...,$T_p$, the acquired gas pressure $P_1$, $P_2$, ...,$P_p$ and the acquired ambient temperature $TA_1$, $TA_2$, $TA_p$,

(iii) update the corrected gas temperature model M so as to minimize a difference between the gas quantities $N_1$, $N_2$, ...,$N_p$ determined for each set of calibration samples $S_1$, $S_2$,...,$S_p$. The method further comprising the following steps during a <u>measurement phase:</u>

(iv) acquiring a gas temperature Tsens measured by the embedded gas temperature sensor 3, a gas pressure Psens measured by the embedded gas pressure sensor 4, and an ambient temperature Tamb measured by an ambient temperature sensor 5 outside the tank 1,

(v) determining a corrected gas temperature Tcor from the corrected gas temperature model M and from the acquired gas temperature Tsens, the acquired gas pressure Psens and the acquired ambient temperature Tamb,

(vi) determining the quantity N of gas from the gas state equation E and from the acquired gas pressure Psens and the determined corrected gas temperature Tcor.

The duration of the calibration phase is considered negligible compared to the expected service life of the switchgear 2. Indeed, an order of magnitude of the duration of the calibration phase is a few hours or a few days, while an order of magnitude of the service life of the switchgear 2 is more than 10 years.

There the tank 1 is considered to be perfectly sealed during the calibration phase. The quantity N of the gas contained in the tank 1 can therefore be considered as constant. All the set of samples $S_1$, $S_2$,..., $S_n$ should in theory provide the same result for the determination of the gas quantity contained in the tank 1, in other words all the quantities $N_1$, $N_2$, ...,$N_n$ should be equal.

In reality, discrepancies between the various gas quantities $N_1$, $N_2$, ..., $N_k$ determined at different instants $t_1$, $t_2$, ..., $t_k$ may happen. These discrepancies come from the fact that the temperature measured by the embedded gas temperature sensor 3 is not fully representative of the actual temperature of the gas contained in the tank 1. Therefore, a representative of the actual temperature of the gas contained in the tank 1. Therefore, a simulated gas temperature, also called corrected gas temperature Tcor can be determined by applying a calculation constraint that the determined gas quantities have to be identical for the different set of samples $S_1, S_2, ..., S_k$ that are acquired during the calibration phase. The corrected gas temperature Tcor is the temperature for which the gas quantity predicted by the gas state equation E is the closest for the various sets of samples $S_1, S_2, ..., S_k$. When the fitting of the corrected gas temperature model is not ideal, corresponding to most practical cases, some residual discrepancies may remain between the gas quantity prediction obtained from each individual set of samples. The gas corrected temperature model M is thus updated by iteration until the best fit is obtained. When the fitting of the corrected gas temperature model is ideal, the gas quantities predicted by the different samples are identical. The discrepancy between the prediction of each sample is in such case null.

Once the model M for determining this corrected gas temperature Tcor has been determined, the same model M is then applied during the measurement phase. Using this model M of corrected temperature Tcor increases the accuracy of the gas quantity calculation compared to a method using only the raw measurements of the different sensors, in particular using only the raw gas temperature. As the model M is extracted from a limited number of experimental data, it is easy to calibrate and can quickly be adapted to any gas tank / sensors configurations. No additional sensor needs to be fitted during the calibration phase. The electrical equipment 100 using the tank 1 doesn't need to be modified in any way.

The measurement phase follows the calibration phase. In other words, the calibration phase is performed before the measurement phase is carried out.

The measurement phase is a monitoring phase that may be carried out throughout the service life of the switchgear.

The measurement phase may be carried out continuously.

The measurement phase may also be carried out at different time intervals. For example, the measurement phase can be performed once every hour, or once every day. Any time periodicity may be used, depending on the available data acquisition resources and data processing resources, and depending upon the expected detection time for ensuring safe operation of the electrical equipment.

The calibration phase may comprise a sub-step of checking of the tank 1 gas tightness. The sub-step is completed before the measurements are acquired. The goal of this preliminary check is to make sure that the tank is properly sealed and that the assumption that the gas quantity does not change during the calibration phase is correct.

The first set of samples $S_1$ corresponds to a first instant $t_1$. The second set of samples $S_2$ corresponds to a second instant $t_2$. The set of samples of rank n $S_n$ corresponds to an instant of rank n $t_n$.

The first instant $t_1$ precedes the second instant $t_2$, which itself precedes the third instant $t_3$, and so on. The first instant $t_1$, the second instant $t_2$, and the third instant $t_3$ come in succession in chronological order. The instant of rank n-1 and the instant of rank n come in succession in chronological order.

The first plurality n of successive sets of calibration samples $S_1, S_2, ..., S_n$ comprises at least three samples. There's no maximum limit to the number of elements in the first plurality n of successive sets of calibration samples $S_1, S_2, ..., S_n$.

Each time of set of samples is acquired, the acquired set of samples comprises the gas pressure P measured by the embedded gas pressure sensor 4, the gas temperature T measured by the embedded gas temperature sensor 3 and the ambient temperature TA measured by an ambient temperature sensor 5 outside the tank 1.

Optionally, other parameters have also be acquired.

Each time of set of samples is acquired, the different samples are acquired simultaneously.

By simultaneously, it is meant that the gas temperature sample, the gas pressure sample and the ambient temperature samples of a given set of samples are acquired within a same time interval whose duration is less than a predetermined threshold. The predetermined threshold is for example 0,5 second.

A shorter threshold, corresponding to a faster sampling frequency, may be employed but is not mandatory as the temporal evolution of the different parameters is relatively slow. Some tests carried out by the applicant have indicated that the time constant of the temperature evolution to be several hours, for example comprised between 8 and 10 hours depending on the internal volume of the tank 1.

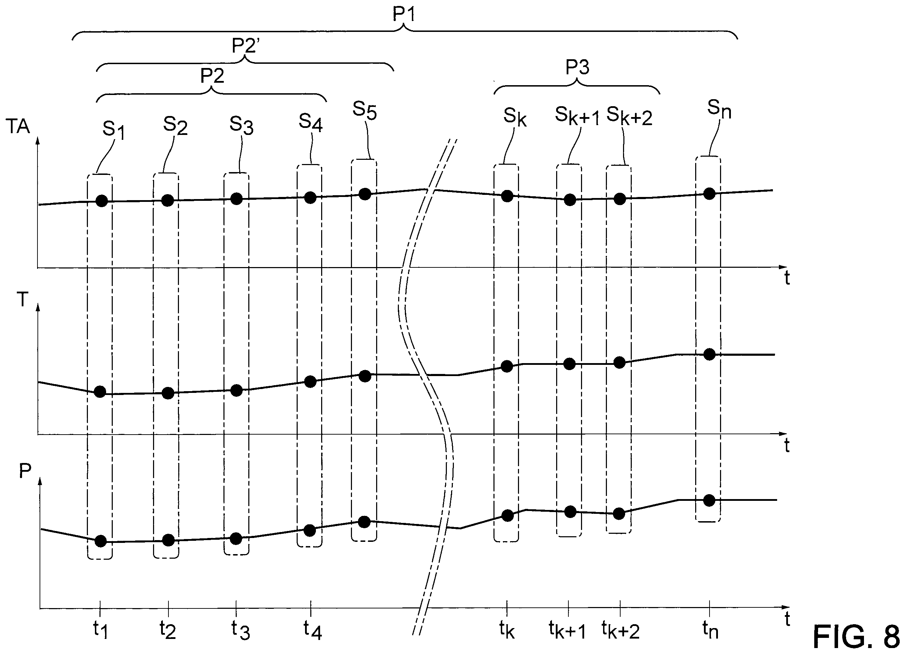

Figure 8 illustrates in a schematic way the acquisition of the set of samples during the calibration phase.

The evolution of gas pressure P, gas temperature T and ambient temperature TA is plotted versus time. Instant $t_1, t_2, ..., t_n$ each correspond to the acquisition of a set of samples, respectively $S_1, S_2, ..., S_n$. Each set of samples comprises a sample of gas pressure, a sample of gas temperature, and a sample of ambient temperature.

The sign P1 refers to the first plurality of successive sets of calibration samples $S_1, S_2, ..., S_n$. The sign P2 refers to the second plurality p of sets of calibration samples $S_1, S_2, ..., S_p$. On the illustrated example, the second plurality of sets of samples comprises 4 sets of samples.

The second plurality p of sets of calibration samples $S_1, S_2, ..., S_p$ is comprised in the first plurality n of sets of calibration samples $S_1, S_2, ..., S_n$.

This means that n sets of samples may be initially acquired, but the gas corrected temperature model M is defined from a smaller number of sets of samples.

For example, n=30 set of samples may be initially acquired, while only 10 set of samples may be used for the definition and update of the gas corrected temperature model M.

In an embodiment, the calibration phase comprises the steps :

(iii-a1) if at step (iii) the difference between the gas quantities $N_1$, $N_2$, ...,$N_p$ determined for each set of calibration samples $S_1$, $S_2$,...,$S_p$ is inferior to a predefined threshold Th, end the calibration phase,

(iii-a2) if at step (iii) the difference between the gas quantity $N_1$, $N_2$, ...,$N_p$ determined for each set of calibration samples $S_1$, $S_2$,...,$S_p$ is superior or equal to the predefined threshold, add one extra set of calibration samples $S_{p+1}$ to the second plurality p of sets of calibration samples $S_1$, $S_2$, ..., $S_p$ to obtain an updated second plurality p+1 of sets of calibration samples $S_1$, $S_2$, ..., $S_p$, $S_{p+1}$ and iterate steps (ii) and (iii) with the updated second plurality p+1 of sets of calibration samples $S_1$, $S_2$, ..., $S_p$, $S_{p+1}$.

The calibration phase ends when the expected accuracy is obtained, ie when the difference between the various gas quantities determined is lower than an acceptance threshold. As long as the expected accuracy is not yet obtained, calibration samples are added to the second plurality of samples, so that more samples are used to calibrate the model M, which helps improving its accuracy. As long as the acceptance threshold is not reached, more calibration set of samples are added to the second plurality in order to improve the model M.

On figure 8, the sign P2 illustrates an initial definition of the second plurality of set of samples used for generating the corrected gas temperature model M, and comprising 4 samples $S_1$, $S_2$, $S_3$, $S_4$.

Assuming the difference between the gas quantity $N_1$, $N_2$, $N_3$, $N_4$ determined for each set of calibration samples $S_1$, $S_2$, $S_3$, $S_4$ is superior or equal to the predefined threshold, a fifth set of samples $S_5$ is integrated into the second plurality of set of samples. The sign P2' illustrates the updated second plurality of sets of samples, with a fifth sample integrated.

In an embodiment, the method comprises the step :

(iii-a3) if at step (iii-a2) the difference between the gas quantity $N_1$, $N_2$, ..., $N_n$ determined for the first plurality n of calibration samples $S_1$, $S_2$,...,$S_n$ is superior or equal to the predefined threshold, iterate step (i) to acquire at least one extra set of calibration samples $S_{n+1}$ and obtain an updated first plurality n+1 of successive sets of calibration samples $S_1$, $S_2$, ..., $S_n$, $S_{n+1}$, and iterate steps (ii) and (iii) to update the corrected gas temperature model M.

As long as the accuracy acceptance threshold is not obtained, extra set of samples are added to the second plurality of sets of samples, until all calibration set of samples of the first plurality has been integrated. The second plurality and the first plurality of set of samples are then identical. If the determined difference is still superior or equal to the predefined acceptance threshold after all calibration samples have been used for calibrating the model M, some extra sets of samples are acquired to continue the calibration process with more data in order to reach the expected accuracy. Alternatively, the calibration process can be restarted from scratch with a completely new set of samples. In that case, the method is applied in the same way as before.

Once the expected accuracy has been obtained, the calibration phase is ended. Nevertheless, a validation may be performed before actually entering in the measurement phase.

For this, the method may comprise between the calibration phase and the measurement phase a validation phase of the corrected gas temperature model M. The validation phase comprises the steps :

(iii-b1) for a third plurality q of sets of calibration samples $S_k$, ..., $S_{k+q}$ comprised in the first plurality n of sets of calibration samples $S_1$, $S_2$, ..., $S_n$, determine a gas quantity $N_k$, ...,$N_{k+q}$ contained in the tank 1 from the gas state equation E and from the corrected gas temperature model M based on the acquired gas temperature $T_k$, ...,$T_{k+q}$, the acquired gas pressure $P_k$, ... ,$P_{k+q}$ and the acquired ambient temperature $TA_k$, ..., $TA_{k+q}$,

(iii-b2) determine a difference between the gas quantities $N_k$, ...,$N_{k+q}$ determined for each set of samples of the third plurality q of sets of calibration samples $S_k$, ..., $S_{k+q}$,

(iii-b3) if the determined difference is inferior to a second predefined threshold Th, authorize entry into the measurement phase.

Some of the sets of samples of the initially acquired data may be used for checking that the associated determined gas quantities are close enough to the expected results, ie for validating the corrected gas temperature model. If the validation is satisfactory, meaning that the experienced discrepancies between the prediction of the various set samples are below an acceptance threshold, a beginning of the measurement phase is authorized.

The third plurality q of sets of calibration samples $S_k$, ..., $S_{k+q}$ may have no common element with the second plurality p of sets of calibration samples $S_1$, $S_2$, ..., $S_p$.

In other words, the model may be calibrated using certain sets of samples, and may be validated using different samples. A same set of samples is not used for both calibration and validation of the gas corrected temperature model.

On figure 8, the sign P3 refers to the third plurality of sets of samples, which is used for validation of the model M and authorization of entry into the measurement phase.

It is also possible that the third plurality of sets of calibration samples and the second plurality of sets of calibration samples overlap each other and have some common elements.

In a non-illustrated example, the initially acquired data comprises 30 sets of samples. The first 12 set of samples are for example used for calibrating the model M. Then the next 8 set of samples are used for validation of the built model. If the discrepancies between the gas quantities calculated for the batch of 8 sets of samples are low enough, the model M is validated, and it is possible to enter into the measurement phase.

In this example, the last 10 samples of the initial batch of 30 sets of samples are not used, since the validation phase gives satisfactory results without using all the available data.

In an embodiment, the method comprises the step:

(iii-b4) if the determined difference is superior or equal to the second predefined threshold Th2, iterate step (i) to acquire at least one extra set of calibration samples $S_{n+1}$ and iterate steps (ii) and (iii) to update the corrected gas temperature model M.

The second predefined threshold Th2 may be higher than the first predefined threshold Th1. In this case, more discrepancies between the determined gas quantities are allowed during the validation phase than during the calibration phase.

The second predefined threshold Th2 may also be equal to the first predefined threshold Th1.

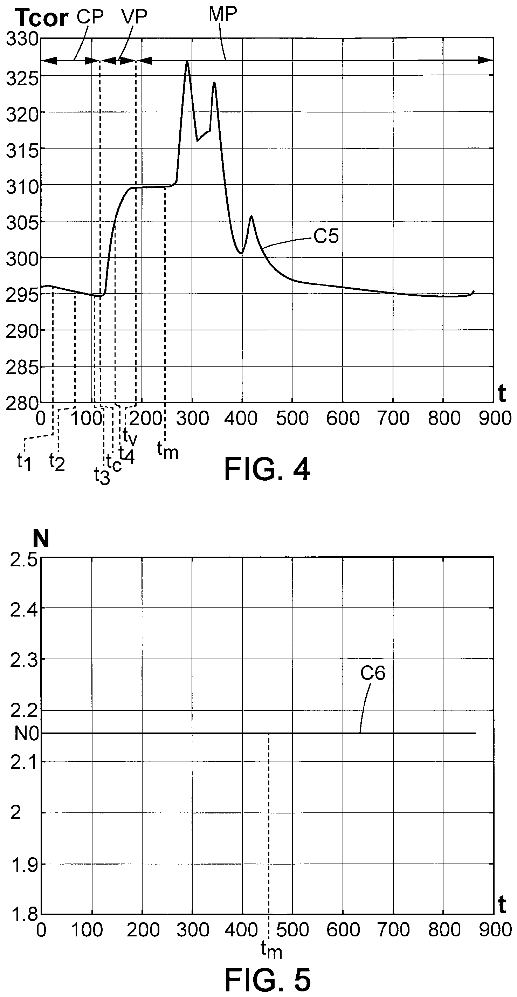

Figure 4 illustrates the sequence of calibration phase, validation phase and measurement phase.

The time period starting at t=0 and ending at t=$t_c$ corresponds to the calibration phase, indicated by the sign CP.

The time period starting at t= $t_c$ and ending at t=$t_v$ corresponds to the validation phase, indicated by the sign VP.

The time period starting at t=$t_v$ corresponds to the measurement phase, indicated by the sign MP. To simplify the figures, only three sets of calibration samples have been represented. The instants $t_1$, $t_2$ and $t_3$ at which each set of calibration samples $S_1$, $S_2$, $S_3$ is respectively acquired are indicated.

The set of samples acquired at instant t= $t_m$ is used for measurement and leakage detection. Curve C5 illustrates the evolution of the corrected gas temperature Tcor when the electrical equipment 100 is operated. The temperature may increase or decrease depending on the evolution of the electrical power and of the ambient temperature.

The volume of the tank 1 is constant.

The quantity N of the gas G encompasses closely related physical parameters that may be the gas number of moles, or the gas mass. It also encompasses the gas density since the internal volume of the tank is kept constant.

At step iii, the model M may be updated so that the different gas quantities $N_1$, $N_2$, ..., $N_p$ determined for each set of samples $S_1$, $S_2$, ..., $S_p$ is the same.

In other words, the difference between the gas quantity $N_1$, $N_2$, ..., $N_p$ determined for each set of samples $S_1$, $S_2$, ..., $S_p$ may be equal to zero.

According to an embodiment of the proposed method, the gas state equation E is the ideal gas law.

The state equation is, for the ideal gas model:

$$P * V = N * r * Tcor$$

(equation 1)

With P the gas pressure, V the gas volume therefore the tank internal volume, N the gas quantity, r the ideal gas constant 8,32 J/mol/K, Tcor the corrected gas temperature in Kelvin. This model is simple to calculate and accurate enough for the conditions in which the electrical equipment is used, with relatively low pressure and temperature.

According to another embodiment of the proposed method, the gas state equation E is a real gas law.

The accuracy may be enhanced with a real gas model.

Many different real gas laws are available and can be used. For example, Van der Waals state equation, or the Virial state equation can be used.

In an embodiment of the method, the corrected temperature model is a multivariate linear regression model.

For example, the corrected gas temperature Tcor is a linear combination of the acquired gas pressure P, of the acquired gas temperature T and of the acquired ambient temperature TA.

$$Tcor = K_0 + K_1 * P + K_2 * T + K_3 * TA$$

(equation 2)

With $K_0$, $K_1$, $K_2$, $K_3$ constant coefficients.

The four coefficients $K_0$, $K_1$, $K_2$, $K_3$ are adjusted by iteration so that the discrepancies between the gas quantities predicted by the different samples are minimized.

When the number of acquired samples is smaller than the number of measured variables, the parameters of such an underdetermined linear system may be derived thanks to the minimum L2 norm solution using, for example, the method of Lagrange multiplier. On the contrary, if the number of samples is larger than the number of measured variables, a classic regression method such as the Least Squares regression method can be selected.

In another embodiment of the method, the corrected temperature model is a perceptron model.

For example, the corrected gas temperature Tcor is a function of a real-valued vector X containing the acquired gas pressure P, the acquired gas temperature T and the acquired ambient temperature TA, of a vector of real-valued weights and of a bias value. The weights and the bias are adjusted using an iterative method such as Stochastic Gradient Descent. The perceptron model can be extended to a multi-layer neural network where the weights and the bias are adjusted using backpropagation.

The acquired gas pressure P, the acquired gas temperature T and the acquired ambient temperature TA are inputs of the perceptron. The corrected temperature Tcor is the output of the perceptron.

In yet another embodiment of the method, the corrected temperature model is a support vector regression.

For example, the corrected Tcor is obtained by mapping the input variables (the acquired gas pressure P, the acquired gas temperature T and the acquired ambient temperature TA) to a high-dimensional space, finding a hyperplane that best fits the data, and then mapping the predicted output back to the original space thanks to computing the dot product between the input vector and the support vectors which consist of a subset of the training data points.

In yet another example of implementation of the method, the corrected temperature model is a gradient boosting regression.

For example, the corrected Tcor is obtained by training a set of decision trees to make predictions. In such a combination of decision trees, each decision tree is added to correct the errors made by the previous decision tree. The process is iterated until the algorithm can no longer improve the model with respect to the predicted output compared to the actual output.

A time interval between two successive sets of calibration samples $S_k$, $S_{k+1}$ of the first plurality n of successive sets of calibration samples $S_1$. $S_2$, ..., $S_n$.

The instant of rank k+1 $t_{k+1}$ and the instant of rank k $t_k$ at offset from each other by a duration longer than one minute, and shorter than one hour.

In an embodiment of the method, the step (i) of acquiring the first plurality n of successive sets of calibration samples $S_1$, $S_2$, ..., $S_n$ may comprise the sub-step :

- after the acquisition of one calibration sample and before the acquisition of the next sample, modifying an electrical current intensity in the switchgear 2 so that the temperature of the gas contained in the tank 1 is modified.

By spreading the values of the different set of samples, the robustness of the model M of determination of corrected gas temperature can be improved, as the measurements are less subject to noise.

On figure 2, curve C3 illustrates the corrected gas temperature Tcor determined from the model M and from the acquired gas temperature Tsens, the acquired gas pressure Psens and the acquired ambient temperature Tamb used as inputs of the model M.

It can be observed that during the time interval ranging from 1500 seconds to 3000 seconds, the corrected gas temperature represented by curve C3 is higher than the curve C2 which an average of different measurements in different locations of the tank, itself higher than curve C1 which is the measurement of the embedded sensor.

The disclosure also relates to a method for detecting a gas leakage in a tank 1 of a gas insulated switchgear 2. The method comprises the steps:

- determining the quantity N of the gas G contained in the tank 1 by the method described above,
- detecting a gas leakage based on the evolution of the gas quantity N in function of time.

The gas leakage detection method may comprise the steps :

- determining an initial quantity N0 of the gas G contained in the tank 1 at the end of the calibration phase,
- determining an updated quantity N of the gas G contained in the tank 1,
- determining a difference D between the determined initial quantity N0 and the determined updated quantity N,
- if the determined difference D is higher than a predetermined threshold Smax, detecting that a leakage is present,
- if the determined difference D is lower than or equal to the predetermined threshold Smax, detecting that the tank 1 is sealed.

The determined difference D can be an absolute difference.

In this case, the predetermined threshold Smax is also an absolute threshold.

The predetermined threshold Smax is a constant value determined with respect to the internal volume of the tank, since the initial quantity of gas filling the tank is proportional to the volume of the tank.

In another embodiment, the determined difference D may be a relative difference. In this case, the predetermined threshold Smax is a relative threshold.

The predetermined threshold Smax is for example comprised between 8 % and 12 %.

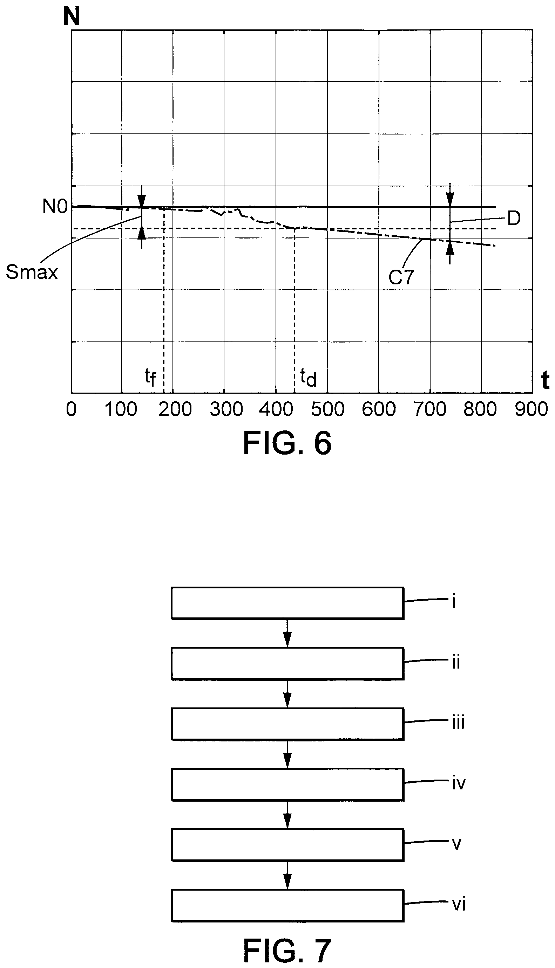

Figures 5 and 6 illustrates the evolution of the gas quantity N versus time t.

The initial gas quantity determined at instant t=0 is indicated by N0 on the vertical axis.

Figure 5 correspond to a gas tight tank, ie with no leakage.

Figure 6 corresponds to a tank initially sealed in which a leakage is initiated.

Curves C6 and C7 indicates the evolution over time of the gas quantity N, continuously updated.

On figure 5, the estimated gas quantity N is constant over time.

On figure 6, the estimated gas quantity N is approximately constant until instant $t=t_f$. From this instant, the estimated gas quantity N, designated by curve C7, gradually drops and keeps deviating from the initial value NO because a leakage is present.

The arrow designated by sign D indicates the deviation between the initial gas quantity N0 represented by an horizontal line and the updated gas quantity N, represented by curve C7. Until instant $t_d$, the difference is small enough and no leakage is detected. At instant $t_d$ the difference D becomes higher than the defined threshold Smax, and a leakage is thus detected.

The threshold Smax is selected large enough to avoid false positive, ie detecting a fault while no actual fault is present. It is also selected small enough to ensure a short response time in case a real fault is present.

The gas leakage detection method may comprise the step:

- emitting a warning signal if a leakage is detected.

The warning signal gives the operators of the electrical equipment an opportunity to investigate and correct the detected fault.

**Claims**

1. A method for determining a quantity (N) of a gas (G) contained in a tank (1) of a gas insulated switchgear (2), the tank (1) comprising an embedded gas temperature sensor (3) and an embedded gas pressure sensor **(4),**
   the method comprising the following steps during a calibration phase:

   (i) acquiring a first plurality (n) of successive sets of calibration samples ($S_1$, $S_2$, ..., $S_n$), each set of calibration samples ($S_1$, $S_2$,..., $S_n$) comprising a gas pressure ($P_1$, $P_2$, ..., $P_n$) measured by the embedded gas pressure sensor (4), a gas temperature ($T_1$, $T_2$, ..., $T_n$) measured by the embedded gas temperature sensor (3) and an ambient temperature ($TA_1$, $TA_2$, ..., $TA_n$) measured by an ambient temperature sensor (5) outside the tank (1),
   (ii) for a second plurality (p) of sets of calibration samples ($S_1$, $S_2$, ..., $S_p$) comprised in the first plurality (n) of sets of calibration samples ($S_1$, $S_2$, ..., $S_n$), determine a gas quantity ($N_1$, $N_2$, ...,$N_p$) contained in the tank (1) from a gas state equation (E) based on the acquired gas pressure ($P_1$, $P_2$, ...,$P_p$), and on a corrected gas temperature model (M) based on the acquired gas temperature ($T_1$, $T_2$, ..., $T_p$), the acquired gas pressure ($P_1$, $P_2$, ...,$P_p$) and the acquired ambient temperature ($TA_1$, $TA_2$, ..., $TA_p$),
   (iii) update the corrected gas temperature model (M) so as to minimize a difference between the gas quantities ($N_1$, $N_2$, ...,$N_p$) determined for each set of calibration samples ($S_1$, $S_2$,...,$S_p$),

   the method further comprising the following steps during a measurement phase:

   (iv) acquiring a gas temperature (Tsens) measured by the embedded gas temperature sensor (3), a gas pressure (Psens) measured by the embedded gas pressure sensor (4), and an ambient temperature (Tamb) measured by an ambient temperature sensor (5) outside the tank (1),
   (v) determining a corrected gas temperature (Tcor) from the corrected gas temperature model (M) and from the acquired gas temperature (Tsens), the acquired gas pressure (Psens) and the acquired ambient temperature (Tamb),
   (vi) determining the quantity (N) of gas from the gas state equation (E) and from the acquired gas pressure (Psens) and the determined corrected gas temperature (Tcor).

2. The method according to claim 1, in which the calibration phase comprises the steps :

   (iii-a1) if at step (iii) the difference between the gas quantity ($N_1$, $N_2$, ...,$N_p$) determined for each set of calibration samples ($S_1$, $S_2$,...,$S_p$) is inferior to a first predefined threshold (Th1), end the calibration phase,

(iii-a2) if at step (iii) the difference between the gas quantity $(N_1, N_2, ..., N_p)$ determined for each set of calibration samples $(S_1, S_2, ..., S_p)$ is superior or equal to the first predefined threshold (Th1), add one extra set of calibration samples $(S_{p+1})$ to the second plurality (p) of sets of calibration samples $(S_1, S_2, ..., S_p)$ to obtain an updated second plurality (p+1) of sets of calibration samples $(S_1, S_2, ..., S_p, S_{p+1})$ and iterate steps (ii) and (iii) with the updated second plurality (p+1) of sets of calibration samples $(S_1, S_2, ..., S_p, S_{p+1})$.

3. The method according to claim 2, comprising the step :
(iii-a3) if at step (iii-a2) the difference between the gas quantity $(N_1, N_2, ..., N_n)$ determined for the first plurality (n) of calibration samples $(S_1, S_2, ..., S_n)$ is superior or equal to the first predefined threshold (Th1), iterate step (i) to acquire at least one extra set of calibration samples $(S_{n+1})$ and obtain an updated first plurality (n+1) of successive sets of calibration samples $(S_1, S_2, ..., S_n, S_{n+1})$, and iterate steps (ii) and (iii) to update the corrected gas temperature model (M).

4. The method according to one of the preceding claims, comprising between the calibration phase and the measurement phase a validation phase of the corrected gas temperature model (M), the validation phase comprising the steps :

(iii-b1) for a third plurality (q) of sets of calibration samples $(S_k, ..., S_{k+q})$ comprised in the first plurality (n) of sets of calibration samples $(S_1, S_2, ..., S_n)$, determine a gas quantity $(N_k, ..., N_{k+q})$ contained in the tank (1) from the gas state equation (E) and from the corrected gas temperature model (M) based on the acquired gas temperature $(T_k, ..., T_{k,q})$, the acquired gas pressure $(P_k, ..., P_{k+q})$ and the acquired ambient temperature $(TA_k, ..., TA_{k+q})$,
(iii-b2) determine a difference between the gas quantities $(N_k, ..., N_{k+q})$ determined for each set of samples of the third plurality (q) of sets of calibration samples $(S_k, ..., S_{k+q})$,
(iii-b3) if the determined difference is inferior to a second predefined threshold (Th2), authorize entry into the measurement phase.

5. The method according to the preceding claim, comprising the step:
(iii-b4) if the determined difference is superior or equal to the second predefined threshold (Th2), iterate step (i) to acquire at least one extra set of calibration samples $(S_{n+1})$ and iterate steps (ii) and (iii) to update the corrected gas temperature model (M).

6. The method according to one of the preceding claims, in which the gas state equation (E) is the ideal gas law.

7. The method according to any of claims 1 to 6, in which the corrected gas temperature model (M) is a multivariate linear regression model.

8. The method according to any of claims 1 to 6, in which the corrected gas temperature model (M) is a perceptron model.

9. The method according to any of claims 1 to 6, in which the corrected gas temperature model (M) is a support vector regression, or a gradient boosting regression.

10. The method according to any of the preceding claims, in which a time interval between two successive sets of calibration samples $(S_k, S_{k+1})$ of the first plurality (n) of successive sets of calibration samples $(S_1, S_2, ..., S_n)$ is more than one minute and less than one hour.

11. The method according to any of the preceding claims, in which the step (i) of acquiring the first plurality (n) of successive sets of calibration samples $(S_1, S_2, ..., S_n)$ comprises the sub-step :

- after the acquisition of one calibration sample and before the acquisition of the next calibration sample, modifying an electrical current intensity in the switchgear (2) so that the temperature of the gas contained in the tank (1) is modified.

12. A method for detecting a gas leakage in a tank (1) of a gas insulated switchgear (2), comprising the steps:

- determining the quantity (N) of the gas (G) contained in the tank (1) by a method according to one of the preceding claims,
- detecting a gas leakage based on the evolution of the gas quantity (N) in function of time.

**13.** The method according to the preceding claim, comprising the steps :

- determining an initial quantity (N0) of the gas (G) contained in the tank (1) at the end of the calibration phase,
- determining an updated quantity (N) of the gas (G) contained in the tank (1) during the measurement phase,
- determining a difference (D) between the determined initial quantity (N0) and the determined updated quantity (N),
- if the determined difference (D) is higher than a maximum threshold (Smax), detecting that a leakage is present,
- if the determined difference (D) is lower than or equal to the maximum threshold (Smax), detecting that the tank (1) is sealed.

**14.** The method according to claim 12 or 13, comprising the step:

- emitting a warning signal if a leakage is detected.

**15.** Electrical equipment (100) comprising a switchgear (2), a tank (1) configured for accommodating the switchgear (2), the tank comprising an embedded gas temperature sensor and an embedded gas pressure sensor, an ambient temperature sensor outside the tank, and an electronic control unit (15) configured for implementing a gas quantity determination method according to any of claims 1 to 11 or for implementing a gas leakage detection method according to any of claims 12 to 14.

**Patentansprüche**

**1.** Verfahren zum Bestimmen einer Menge (N) eines Gases (G), welches in einem Behälter (1) einer gasisolierten Schaltanlage (2) enthalten ist, wobei der Behälter (1) einen integrierten Gastemperatursensor (3) und einen integrierten Gasdrucksensor (4) umfasst,

wobei das Verfahren die folgenden Schritte während einer Kalibrierungsphase umfasst:

(i) Erlangen einer ersten Mehrzahl (n) von aufeinanderfolgenden Sätzen von Kalibrierungsproben $(S_1, S_2, ..., S_n)$, wobei jeder Satz von Kalibrierungsproben $(S_1, S_2, ..., S_n)$ einen Gasdruck $(P_1, P_2, ..., P_n)$, welcher durch den integrierten Gasdrucksensor (4) gemessen wird, eine Gastemperatur $(T_1, T_2, ..., T_n)$, welche durch den integrierten Gastemperatursensor (3) gemessen wird, und eine Umgebungstemperatur $(TA_1, TA_2, ..., T_n)$ umfasst, welche durch einen Umgebungstemperatursensor (5) außerhalb des Behälters (1) gemessen wird,

(ii) für eine zweite Mehrzahl (p) von Sätzen von Kalibrierungsproben $(S_1, S_2, ..., S_p)$, welche in der ersten Mehrzahl (n) von Sätzen von Kalibrierungsproben $(S_1, S_2, ..., S_n)$ umfasst sind, Bestimmen einer Gasmenge $(N_1, N_2, ..., N_p)$, welche in dem Behälter (1) enthalten ist, aus einer Gaszustandsgleichung (E), basierend auf dem erlangten Gasdruck $(P_1, P_2, ..., P_p)$ und auf einem korrigierten Gastemperaturmodell (M) basierend auf der erlangten Gastemperatur $(T_1, T_2, ..., T_p)$, dem erlangten Gasdruck $(P_1, P_2, ..., P_p)$ und der erlangten Umgebungstemperatur $(TA_1, TA_2, ..., TA_p)$,

(iii) Aktualisieren des korrigierten Gastemperaturmodells (M), um eine Differenz zwischen den Gasmengen $(N_1, N_2, ..., N_p)$, welche für jeden aus der zweiten Mehrzahl von Sätzen von Kalibrierungsproben $(S_1, S_2, ..., S_p)$ bestimmt werden, zu minimieren,

wobei das Verfahren ferner die folgenden Schritte während einer Messphase umfasst:

(iv) Erlangen einer Gastemperatur (Tsens), welche durch den integrierten Gastemperatursensor (3) gemessen wird, eines Gasdrucks (Psens), welcher durch den integrierten Gasdrucksensor (4) gemessen wird, und einer Umgebungstemperatur (Tamb), welche durch einen Umgebungstemperatursensor (5) außerhalb des Behälters (1) gemessen wird,

(v) Bestimmen einer korrigierten Gastemperatur (Tcor) aus dem korrigierten Gastemperaturmodell (M) und aus der erlangten Gastemperatur (Tsens), dem erlangten Gasdruck (Psens) und der erlangten Umgebungstemperatur (Tamb),

(vi) Bestimmen der Menge (N) von Gas aus der Gaszustandsgleichung (E) und aus dem erlangten Gasdruck (Psens) und der bestimmten korrigierten Gastemperatur (Tcor).

**2.** Verfahren nach Anspruch 1, in welchem die Kalibrierungsphase die Schritte umfasst:

(iii-a1) wenn bei Schritt (iii) die Differenz zwischen der Gasmenge ($N_1$, $N_2$, ..., $N_p$), welche für jeden Satz von Kalibrierungsproben ($S_1$, $S_2$, ..., $S_p$) bestimmt worden ist, kleiner als ein erste vorbestimmter Schwellenwert (Th1) ist, Beenden der Kalibrierungsphase,

(iii-a2) wenn bei Schritt (iii) die Differenz zwischen der Gasmenge ($N_1$, $N_2$, ..., $N_p$), welche für jeden Satz von Kalibrierungsproben ($S_1$, $S_2$, ..., $S_p$) bestimmt worden ist, größer als oder gleich wie der erste vorbestimmte Schwellenwert (Th1) ist, Ergänzen eines zusätzlichen Satzes von Kalibrierungsproben ($S_{p+1}$) zu der zweiten Mehrzahl (p) von Sätzen von Kalibrierungsproben ($S_1$, $S_2$, ..., $S_p$), um eine aktualisierte zweite Mehrzahl (p+1) von Sätzen von Kalibrierungsproben ($S_1$, $S_2$, ..., $S_p$, $S_{p+1}$) zu erhalten und Iterieren der Schritte (ii) und (iii) mit der aktualisierten zweiten Mehrzahl (p+1) von Sätzen von Kalibrierungsproben ($S_1$, $S_2$, ..., $S_p$, $S_{p+1}$).

3. Verfahren nach Anspruch 2, umfassend den Schritt:
(iii-a3), wenn bei Schritt (iii-a2) die Differenz zwischen der Gasmenge ($N_1$, $N_2$, ..., $N_n$), welche für die erste Mehrzahl (n) von Kalibrierungsproben ($S_1$, $S_2$, ..., $S_n$) bestimmt worden ist, größer als oder gleich wie der erste vorbestimmte Schwellenwert (Th1) ist, Iterieren des Schritts (i) um wenigstens einen zusätzlichen Satz von Kalibrierungsproben ($S_{n+1}$) zu erlangen und Erhalten einer aktualisierten ersten Mehrzahl (n+1) von aufeinanderfolgenden Sätzen von Kalibrierungsproben ($S_1$, $S_2$, ..., $S_n$, $S_{n+1}$), und Iterieren der Schritte (ii) und (iii) um das korrigierte Gastemperaturmodell (M) zu aktualisieren.

4. Verfahren nach einem der vorhergehenden Ansprüche, umfassend eine Validierungsphase des korrigierten Gastemperaturmodells (M) zwischen der Kalibrierungsphase und der Messphase, wobei die Validierungsphase die Schritte umfasst:

(iii-b1) für eine dritte Mehrzahl (q) von Sätzen von Kalibrierungsproben ($S_k$, ..., $S_{k+q}$), welche in der ersten Mehrzahl (n) von Sätzen von Kalibrierungsproben ($S_1$, $S_2$, ..., $S_n$) umfasst sind, Bestimmen einer Gasmenge ($N_k$, ..., $N_{k+q}$), welche in dem Behälter (1) beinhaltet ist, aus der Gaszustandsgleichung (E) und aus dem korrigierten Gastemperaturmodell (M) basierend auf der erlangten Gastemperatur ($T_k$, ..., $T_{k+q}$), dem erlangten Gasdruck ($P_k$, ..., $P_{k+q}$) und der erlangten Umgebungstemperatur ($TA_k$, ..., $TA_{k+q}$),
(iii-b2) Bestimmen einer Differenz zwischen den Gasmengen ($N_k$, ..., $N_{k+q}$), welche für jeden Satz von Proben der dritten Mehrzahl (q) von Sätzen von Kalibrierungsproben ($S_k$, ..., $S_{k+q}$) bestimmt wurden,
(iii-b3) wenn die bestimmte Differenz kleiner als ein zweiter vorbestimmter Schwellenwert (Th2) ist, autorisieren eines Eintretens in die Messphase.

5. Verfahren nach dem vorhergehenden Anspruch, umfassend den Schritt:
(iii-b4), wenn die bestimmte Differenz größer oder gleich wie der zweite vorbestimmte Schwellenwert (Th2) ist, Iterieren des Schritts (i), um wenigstens einen zusätzlichen Satz von Kalibrierungsproben ($S_{n+1}$) zu erlangen und Iterieren der Schritte (ii) und (iii) um das korrigierte Gastemperaturmodell (M) zu aktualisieren.

6. Verfahren nach einem der vorhergehenden Ansprüche, wobei die Gaszustandsgleichung (E) das ideale Gasgesetz ist.

7. Verfahren nach einem der Ansprüche 1 bis 6, wobei das korrigierte Gastemperaturmodell (M) ein multivariates lineares Regressionsmodell ist.

8. Verfahren nach einem der Ansprüche 1 bis 6, wobei das korrigierte Gastemperaturmodell (M) ein Perzeptronmodell ist.

9. Verfahren nach einem der Ansprüche 1 bis 6, wobei das korrigierte Gastemperaturmodell (M) eine Support-Vector-Regression oder eine gradientenverstärkende Regression ist.

10. Verfahren nach einem der vorhergehenden Ansprüche, wobei ein Zeitintervall zwischen zwei aufeinanderfolgenden Sätzen von Kalibrierungsproben ($S_k$, $S_{k+1}$) der ersten Mehrzahl (n) von aufeinanderfolgenden Sätzen von Kalibrierungsproben ($S_1$, $S_2$, ..., $S_n$) mehr als eine Minute und weniger als eine Stunde beträgt.

11. Verfahren nach einem der vorhergehenden Ansprüche, wobei der Schritt (i) eines Erlangens der ersten Mehrzahl (n) von aufeinanderfolgenden Sätzen von Kalibrierungsproben ($S_1$, $S_2$, ..., $S_n$) den Teilschritt umfasst:

- nach dem Erlangen einer Kalibrierungsprobe und vor dem Erlangen der nächsten Kalibrierungsprobe, Modifizieren einer Intensität eines elektrischen Stroms in der Schaltanlage (2), sodass die Temperatur des in dem Behälter (1) beinhalteten Gases modifiziert wird.

12. Verfahren zum Detektieren eines Gaslecks in einem Behälter (1) einer gasisolierten Schaltanlage (2), umfassend die Schritte:

- Bestimmen der Menge (N) des in dem Behälter (1) beinhalteten Gases (G) durch ein Verfahren nach einem der vorhergehenden Ansprüche,
- Detektieren eines Gaslecks basierend auf der Entwicklung der Gasmenge (N) als Funktion der Zeit.

13. Verfahren nach dem vorhergehenden Anspruch, umfassend die Schritte:

- Bestimmen einer initialen Menge (N0) des in dem Behälter (1) beinhalteten Gases (G) an dem Ende der Kalibrierungsphase,
- Bestimmen einer aktualisierten Menge (N) des in dem Behälter (1) beinhalteten Gases (G) während der Messphase,
- Bestimmen einer Differenz (D) zwischen der bestimmten initialen Menge (N0) und der bestimmten aktualisierten Menge (N),
- wenn die bestimmte Differenz (D) größer als ein maximaler Schwellenwert (Smax) ist, Detektieren, dass ein Leck vorliegt,
- wenn die bestimmte Differenz (D) kleiner als oder gleich wie der maximale Schwellenwert (Smax) ist, Detektieren, dass der Behälter (1) abgedichtet ist.

14. Verfahren nach Anspruch 12 oder 13, umfassend den Schritt:

- Ausgeben eines Warnsignals, wenn ein Leck detektiert worden ist.

15. Elektrische Vorrichtung (100), umfassend eine Schaltanlage (2), einen Behälter (1), welcher dazu eingerichtet ist, die Schaltanlage (2) aufzunehmen, wobei der Behälter einen integrierten Gastemperatursensor und einen integrierten Gasdrucksensor, einen Umgebungstemperatursensor außerhalb des Behälters und eine elektronische Steuerungseinheit (15) umfasst, welche zur Implementierung eines Gasmengenbestimmungsverfahrens nach einem der Ansprüche 1 bis 11 oder zur Implementierung eines Gasleckdetektionsverfahrens nach einem der Ansprüche 12 bis 14 eingerichtet ist.

## Revendications

1. Procédé pour déterminer une quantité (N) d'un gaz (G) contenu dans une cuve (1) d'un appareil de commutation (2) à isolation gazeuse, la cuve (1) comprenant un capteur de température de gaz intégré (3) et un capteur de pression de gaz intégré (4), le procédé comprenant les étapes suivantes durant une phase d'étalonnage :

(i) l'acquisition d'une première pluralité (n) d'ensembles successifs d'échantillons d'étalonnage ($S_1$, $S_2$, ..., $S_n$), chaque ensemble d'échantillons d'étalonnage ($S_1$, $S_2$, ..., $S_n$) comprenant une pression de gaz ($P_1$, $P_2$, ..., $P_n$) mesurée par le capteur de pression de gaz intégré (4), une température de gaz ($T_1$, $T_2$, ..., $T_n$) mesurée par le capteur de température de gaz intégré (3) et une température ambiante ($TA_1$, $TA_2$, ..., $TA_n$) mesurée par un capteur de température ambiante (5) à l'extérieur du réservoir (1),
(ii) pour une deuxième pluralité (p) d'ensembles d'échantillons d'étalonnage ($S_1$, $S_2$, ..., $S_p$) compris dans la première pluralité (n) d'ensembles d'échantillons d'étalonnage ($S_1$, $S_2$, ..., $S_n$), la détermination d'une quantité ($N_1$, $N_2$, ..., Np) de gaz contenue dans le réservoir (1) à partir d'une équation d'état de gaz (E) basée sur la pression de gaz ($P_1$, $P_2$, ..., $P_p$) acquise, et sur un modèle de température de gaz corrigée (M) basé sur la température de gaz ($T_1$, $T_2$, ..., $T_p$) acquise, la pression de gaz ($P_1$, $P_2$, ..., $P_p$) acquise et la température ambiante ($TA_1$, $TA_2$, ..., $TA_p$) acquise,
(iii) la mise à jour du modèle de température de gaz corrigée (M) de manière à minimiser une différence entre les quantités ($N_1$, $N_2$, ..., $N_p$) de gaz déterminées pour chacun de ladite deuxième pluralité d'ensembles d'échantillons d'étalonnage ($S_1$, $S_2$, ..., $S_p$),

le procédé comprenant en outre les étapes suivantes durant une phase de mesure :

(iv) l'acquisition d'une température de gaz (Tsens) mesurée par le capteur de température de gaz intégré (3), d'une pression de gaz (Psens) mesurée par le capteur de pression de gaz intégré (4), et d'une température ambiante (Tamb) mesurée par un capteur de température ambiante (5) à l'extérieur du réservoir (1),

(v) la détermination d'une température de gaz corrigée (Tcor) à partir du modèle de température de gaz corrigée (M) et à partir de la température de gaz (Tsens) acquise, de la pression de gaz (Psens) acquise et de la température ambiante (Tamb) acquise,

(vi) la détermination de la quantité (N) de gaz à partir de l'équation d'état du gaz (E) et à partir de la pression de gaz (Psens) acquise et de la température de gaz corrigée (Tcor) déterminée.

2. Procédé selon la revendication 1, dans lequel la phase d'étalonnage comprend les étapes :

(iii-al) si à l'étape (iii) la différence entre la quantité $(N_1, N_2, ..., N_p)$ de gaz déterminée pour chaque ensemble d'échantillons d'étalonnage $(S_1, S_2, ..., S_p)$ est inférieure à un premier seuil prédéfini (Th1), mettre fin à la phase d'étalonnage,

(iii-a2) si à l'étape (iii) la différence entre la quantité $(N_1, N_2, ..., N_p)$ de gaz déterminée pour chaque ensemble d'échantillons d'étalonnage $(S_1, S_2, ..., S_p)$ est supérieure ou égale au premier seuil prédéfini (Th1), ajouter un ensemble d'échantillons d'étalonnage supplémentaire $(S_{p+1})$ à la deuxième pluralité (p) d'ensembles d'échantillons d'étalonnage $(S_1, S_2, ..., S_p)$ pour obtenir une seconde pluralité mise à jour (p+1) d'ensembles d'échantillons d'étalonnage $(S_1, S_2, ..., S_p, S_{p+1})$ et répéter les étapes (ii) et (iii) avec la seconde pluralité mise à jour (p+1) d'ensembles d'échantillons d'étalonnage $(S_1, S_2, ..., S_p, S_{p+1})$.

3. Procédé selon la revendication 2, comprenant l'étape :
(iii-a3) si à l'étape (iii-a2) la différence entre la quantité $(N_1, N_2, ..., N_n)$ de gaz déterminée pour la première pluralité (n) d'échantillons d'étalonnage $(S_1, S_2, ..., S_n)$ est supérieure ou égale au premier seuil prédéfini (Th1), répéter l'étape (i) pour acquérir au moins un ensemble d'échantillons d'étalonnage supplémentaire $(S_{n+1})$ et obtenir une première pluralité mise à jour (n+1) d'ensembles d'échantillons d'étalonnage $(S_1, S_2, ..., S_n, S_{n+1})$ successifs, et répéter les étapes (ii) et (iii) pour mettre à jour le modèle de température de gaz corrigée (M).

4. Procédé selon l'une des revendications précédentes, comprenant entre la phase d'étalonnage et la phase de mesure une phase de validation du modèle de température de gaz corrigée (M), la phase de validation comprenant les étapes :

(iii-b1) pour une troisième pluralité (q) d'ensembles d'échantillons d'étalonnage $(S_k, ..., S_{k+q})$ compris dans la première pluralité (n) d'ensembles d'échantillons d'étalonnage $(S_1, S_2, ..., S_n)$, déterminer une quantité $(N_k, ..., N_{k+q})$ de gaz contenue dans le réservoir (1) à partir de l'équation d'état du gaz (E) et à partir du modèle de température de gaz corrigée (M) basé sur la température de gaz $(T_k, ..., T_{k+q})$ acquise, la pression de gaz $(P_k, ..., P_{k+q})$ acquise et la température ambiante $(TA_k, ..., TA_{k+q})$ acquise,

(iii-b2) déterminer une différence entre les quantités $(N_k, ..., N_{k+q})$ de gaz déterminées pour chaque ensemble d'échantillons de la troisième pluralité (q) d'ensembles d'échantillons d'étalonnage $(S_k, ..., S_{k+q})$,

(iii-b3) si la différence déterminée est inférieure à un second seuil prédéfini (Th2), autoriser l'entrée dans la phase de mesure.

5. Procédé selon la revendication précédente, comprenant l'étape :
(iii-b4) si la différence déterminée est supérieure ou égale au second seuil prédéfini (Th2), répéter l'étape (i) pour acquérir au moins un ensemble d'échantillons d'étalonnage supplémentaire $(S_{n+1})$ et répéter les étapes (ii) et (iii) pour mettre à jour le modèle de température de gaz corrigée (M).

6. Procédé selon l'une des revendications précédentes, dans lequel l'équation d'état du gaz (E) est la loi des gaz parfaits.

7. Procédé selon l'une quelconque des revendications 1 à 6, dans lequel le modèle de température de gaz corrigée (M) est un modèle de régression linéaire à plusieurs variables.

8. Procédé selon l'une quelconque des revendications 1 à 6, dans lequel le modèle de température de gaz corrigée (M) est un modèle de perceptron.

9. Procédé selon l'une quelconque des revendications 1 à 6, dans lequel le modèle de température de gaz corrigée (M) est une régression à vecteurs de support, ou une régression par gradient boosting.

**10.** Procédé selon l'une quelconque des revendications précédentes, dans lequel un intervalle de temps entre deux ensembles d'échantillons d'étalonnage ($S_k$, $S_{k+1}$) successifs de la première pluralité ($n$) d'ensembles d'échantillons d'étalonnage ($S_1$, $S_2$, ..., $S_n$) successifs est de plus d'une minute et de moins d'une heure.

**11.** Procédé selon l'une quelconque des revendications précédentes, dans lequel l'étape (i) consistant à acquérir la première pluralité ($n$) d'ensembles d'échantillons d'étalonnage ($S_1$, $S_2$, ..., $S_n$) successifs comprend la sous-étape :

- après l'acquisition d'un échantillon d'étalonnage et avant l'acquisition de l'échantillon d'étalonnage suivant, la modification d'une intensité de courant électrique dans l'appareil de commutation (2) à isolation gazeuse de façon à ce que la température du gaz contenu dans le réservoir (1) soit modifiée.

**12.** Procédé pour détecter une fuite de gaz dans un réservoir (1) d'un appareil de commutation (2) à isolation gazeuse, comprenant les étapes :

- la détermination de la quantité ($N$) du gaz ($G$) contenu dans le réservoir (1) par un procédé selon l'une des revendications précédentes,
- la détection d'une fuite de gaz sur la base de l'évolution de la quantité ($N$) de gaz en fonction du temps.

**13.** Procédé selon la revendication précédente, comprenant les étapes :

- la détermination d'une quantité initiale ($N0$) du gaz ($G$) contenu dans le réservoir (1) à la fin de la phase d'étalonnage,
- la détermination d'une quantité ($N$) mise à jour du gaz ($G$) contenu dans le réservoir (1) durant la phase de mesure,
- la détermination d'une différence ($D$) entre la quantité initiale ($N0$) déterminée et la quantité ($N$) mise à jour déterminée,
- si la différence ($D$) déterminée est supérieure à un seuil maximal ($Smax$), le fait de détecter qu'une fuite est présente,
- si la différence ($D$) déterminée est inférieure ou égale au seuil maximal ($Smax$), le fait de détecter que le réservoir (1) est étanche.

**14.** Procédé selon la revendication 12 ou 13, comprenant l'étape :

- l'émission d'un signal d'avertissement si une fuite est détectée.

**15.** Équipement électrique (100) comprenant un appareil de commutation (2) à isolation gazeuse, un réservoir (1) configuré pour recevoir l'appareil de commutation (2) à isolation gazeuse, le réservoir comprenant un capteur de température de gaz intégré et un capteur de pression de gaz intégré, un capteur de température ambiante à l'extérieur du réservoir, et une unité électronique de commande (15) configuré pour mettre en œuvre un procédé de détermination de quantité de gaz selon l'une quelconque des revendications 1 à 11 ou pour mettre en œuvre un procédé de détection de fuite de gaz selon l'une quelconque des revendications 12 à 14.

FIG. 1

EP 4 462 095 B1

FIG. 2

FIG. 3

FIG. 4

FIG. 5

FIG. 6

FIG. 7

TA

T

P

$P1$ $P2$ $P2'$ $P3$

$S_1$ $S_2$ $S_3$ $S_4$ $S_5$ $S_k$ $S_{k+1}$ $S_{k+2}$ $S_n$

$t_1$ $t_2$ $t_3$ $t_4$ $t_k$ $t_{k+1}$ $t_{k+2}$ $t_n$

$t$

FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015308938 A1
- JP H08105813 A
- US 2017030799 A1